# EUROPÄISCHE PATENTSCHRIFT

(11) **EP 0 659 739 B1**
(45) Veröffentlichungstag und Bekanntmachung des Hinweises auf die Patenterteilung: **24.09.1997**
(21) Anmeldenummer: 94119704.8
(22) Anmeldetag: 14.12.1994
(51) Int. Cl.: C07C 403/20, C07F 9/54

(54) **Verfahren zur Herstellung von 9-(Z)-Retinsäure**
Process for the preparation of 9-(Z) retinoic acid
Procédé pour la préparation de l'acide 9-(Z)-rétinoique

(30) Priorität: 23.12.1993 DE 4344148
(43) Veröffentlichungstag der Anmeldung: 28.06.1995
(73) Patentinhaber: BASF Aktiengesellschaft, 67063 Ludwigshafen (DE)
(72) Erfinder: John, Dr. Michael, D-67061 Ludwigshafen (DE); Paust, Dr. Joachim, D-67141 Neuhofen (DE)

(56) Entgegenhaltungen:
- EP-A- 0 000 140
- ANGEWANDTE CHEMIE, Bd.72, Nr.22, 1960, Weinheim, DE, Seiten 811 - 819, H. POMMER: "Synthesen in der Vitamin-A-Reihe"

## Beschreibung

9-(Z)-Retinsäure ist eine physiologisch bedeutsame Substanz (vgl. Nature 355 (1992) Seiten 359 - 361). Sie besitzt eine hohe Bindungsaffinität gegenüber dem Kern-Retinsäureacceptor RXR_{α}, der durch die direkte Ligandeninteraktion aktiviert wird. Als Resultat dieser Wechselwirkung wird eine veränderte Genexpression beobachtet, die eine weitergehende Beeinflussung von Zellprozessen ermöglicht.

Da bisher kein brauchbares Verfahren für die Herstellung von 9-(Z)-Retinsäure bekannt ist, war es Aufgabe der Erfindung, ein vorteilhaftes Verfahren zu dessen Herstellung zu entwickeln.

Das bisher am häufigsten verwendete Retinsäure-Isomere, die all-(E)-Retinsäure, wird vorteilhaft durch Wittig-Reaktion eines β-Jonylidenethyl-triphenylphosphoniumsalzes (C₁₅-Triphenylphosphoniumsalzes) mit einem β-Formyl-crotonsäureester hergestellt. Eine nachfolgende Trennung der resultierenden Doppelbindungsisomeren ist nur äußerst aufwendig unter Anwendung präparativer chromatographischer Methoden durchführbar.

Bei der Herstellung des für technische Vitamin-A-Synthesen sowie für die Herstellung von anderen Vitamin-A-Abkömmlingen, wie Retinal und der Retinsäure, benötigten C₁₅-Triphenylphosphoniumsalzes (siehe z.B. H. Pommer et al. in Angew. Chem. 77 (1965) 277 - 360) fällt nach Abtrennung des Wertproduktes eine Mutterlauge an, in der neben all-(E)-C₁₅-Triphenylphosphoniumsalz das 9-(Z)-Isomere in einem Anteil von 10 bis 60 Gew.-%, insbesondere 30 bis 55 Gew.-%, bezogen auf Gesamt-C₁₅-Triphenylphosphoniumsalz, enthälten ist. Eine direkte Verwendung dieser Mutterlauge für die Herstellung von 9-(Z)-Retinsäure ist nicht ohne große Schwierigkeiten möglich.

Es war daher die Aufgabe der Erfindung ein Verfahren zu entwikkeln mit dessen Hilfe die gewünschte 9-(Z)-Retinsäure auf einfache Weise aus den Mutterlaugen der C₁₅-Triphenylphosphoniumsalzgewinnung hergestellt werden kann.

Gegenstand der Erfindung ist ein Verfahren zur Herstellung von 9-(Z)-Retinsäure aus Mutterlaugen der technischen Herstellung von C₁₅-Triarylphosphoniumsalzen der allgemeinen Formel I 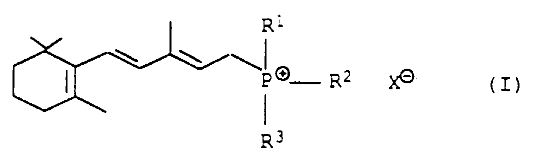 in der R¹ bis R³ für einen Arylrest, vorzugsweise einen Phenylrest, stehen und X für Halogen oder (HSO₄) steht, in einem organischen Lösungsmittel, das dadurch gekennzeichnet ist, daß man
A. in den aus den Mutterlaugen durch Extraktion mit Wasser und Einengen der wäßrigen Phase isolierten C₁₅-Triarylphosphoniumsalzen den Anteil an 9-(Z)-C₁₅-Triarylphosphoniumsalzen dadurch anreichert, daß man das ölige C₁₅-Triaryl-phosphoniumsalzgemisch unter Erwärmen in möglichst wenig von einem niederen Alkanol, vorzugsweise in Isopropanol, löst und das beim Abkühlen auskristallisierende all-(E)-C₁₅-Triarylphosphoniumsalz abtrennt,
B. das erhaltene, an dem 9-(Z)-Isomeren angereicherte C₁₅-Triarylphosphoniumsalz der Formel I in Gegenwart einer bei Wittig-Reaktionen üblicherweise verwendeten Base, insbesondere in Gegenwart eines Alkali- oder Erdalkalihydroxids, eines Alkali- oder Erdalkaliamids, eines Alkalicarbonats oder von Ammoniak in einem für Wittig-Reaktionen geeigneten Lösungsmittel mit einem β-Formyl-crotonsäurealkylester der allgemeinen Formel II 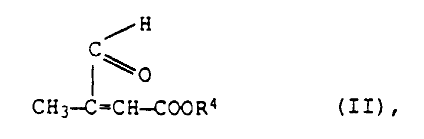 in der R⁴ für eine C₁-C₄-Alkylgruppe steht, umsetzt und
C. das bei der für Wittig-Reaktionen üblichen Aufarbeitung erhaltene obige Retinsäureestergemisch in einem C₃- bis C₉-Alkanol, vorzugsweise in einem Propanol oder einem Butanol, insbesondere in Isobutanol, verseift und die erhaltene 9-(Z)-Retinsäure aus der alkanolischen, vorzugsweise propanolischen oder butanolischen Lösung ggf. durch Zusatz von Methanol kristallin ausfällt, wobei die gebildete all-(E)-Retinsäure und andere unbekannte Retinsäureisomere in der alkanolischen Lösung verbleiben.

Besonders reine 9-(Z)-Retinsäure erhält man, wenn man die erhaltene 9-(Z)-Retinsäure anschließend aus einem Butanol/Methanol-Gemisch, vorzugsweise einem Isobutanol/Methanol-Gemisch kristallisiert.

Mit besonderem Vorteil gelingt das erfindungsgemäße Verfahren, wenn man im Reaktionsschritt A
den Anteil an dem 9-(Z)-C₁₅-Triarylphosphoniumsalz aus der Mutterlauge der C₁₅-Triarylphosphoniumsalz-Herstellung dadurch isoliert und anreichert, daß man
a) die Mutterlauge mit Wasser versetzt und die organische Phase abtrennt,
b) die wäßrige Phase unter schonenden Bedingungen einengt,
c) das so erhaltene Öl unter Erwärmen in möglichst wenig Isopropanol löst,
d) das durch Abkühlen auskristallisierende all-(E)-C₁₅-Triarylphosphoniumsalz abtrennt und
e) die erhaltene isopropanolische Lösung unter schonenden Bedingungen einengt.

Die Herstellung der C₁₅-Triarylphosphoniumsalze erfolgt in an sich bekannter Weise im allgemeinen in Lösungsmitteln, wie Toluol, Acetonitril, Xylol, Decalin, Methylenchlorid, Methyl-tert.-butylether, Wasser und Alkanolen.

Dementsprechend bestehen die für das erfindungsgemäße Verfahren benötigten Mutterlaugen aus einem dieser Lösungsmittel oder einem Lösungsmittelgemisch, Triphenylphosphinoxid, Halogenwasserstoffsäure bzw. Schwefelsäure und C₁₅-Kohlenwasserstoff sowie phosphoniumsalzen der Halogenwasserstoffsäure bzw. Schwefelsäure.

Sie enthalten - je nach den Reaktionsbedingungen - das 9-(Z)-Isomere in einem Anteil von 10 bis 60 %, vorzugsweise 30 bis 55 %, bezogen auf die Gesamt-C₁₅-Triarylphosphoniumsalzmenge. Aus dieser Mutterlauge werden die C₁₅-Triarylphosphoniumsalze mit Wasser extrahiert und die wäßrige Phase unter schonenden Bedingungen eingeengt. Das so erhaltene Öl wird in soviel von einem niederen Alkanol, vorzugsweise in soviel Isopropanol, aufgenommen, daß eine Konzentration des Salzes von 30 bis 70 Gew.-%, vorzugsweise 40 bis 60 Gew.-%, insbesondere 45 bis 55 Gew.-% vorliegt. Anschließend läßt man die erhaltene Lösung bei Temperaturen von -50 bis 25°C, vorzugsweise -30 bis 0°C stehen, wobei all-(E)-Triarylphosphoniumsalz auskristallisiert und so abgetrennt werden kann. Nach dieser Anreicherung des 9-(Z)-C₁₅-Salzes liegt in der Mutterlauge ein Verhältnis von 9-(Z)- zu all-(E)-C₁₅-Triphenylphosphoniumsalz von etwa 1:1 bis zu 50:1 vor. Gemäß dem Ausführungsbeispiel wurde ein Verhältnis von 90:6, entsprechend etwa 15:1 erzielt. Eine weitere Anreicherung des 9-(Z)-Isomeren ist auf Basis einer kinetischen Kontrolle der anschließenden Wittig-Reaktion möglich, da das verbliebene all-(E)-Isomere sehr viel schneller die Wittig-Reaktion eingeht als das 9-(Z)-C₁₅-Triarylphosphoniumsalz. Wird nämlich das C₁₅-Triarylphosphoniumsalz-Gemisch in einer Wittig-Reaktion mit einer dem noch enthaltenen Anteil an all-(E)-C₁₅-Salz entsprechenden Menge einer Aldehydverbindung umgesetzt, so gestattet eine saure Aufarbeitung des Reaktionsansatzes die Isolierung von einem 9-(Z)-C₁₅-Triarylphosphoniumsalz aufgrund der kinetischen Differenzierung.

Einen weiteren Vorteil weist das erfindungsgemäße Verfahren zur Herstellung von 9-(Z)-Retinsäure auf, wenn man im Reaktionsschritt B
das an dem 9-(Z)-Isomeren angereicherte C₁₅-Triarylphosphoniumsalz der allgemeinen Formel I in Gegenwart einer bei Wittig-Reaktionen üblicherweise verwendeten Base, insbesondere in Gegenwart eines Alkali- oder Erdalkalihydroxids, eines Alkalicarbonats oder von Ammoniak in Dimethylformamid, einem Alkanol-Dimethylformamid-Gemisch, in einem Alkanol-Wasser-Gemisch, in 1,4-Dioxan, Wasser oder in N-Methyl-Pyrrolidon, vorzugsweise in Dimethylformamid, als Lösungsmittel mit dem β-Formyl-crotonsäurealkylester der allgemeinen Formel II umsetzt. Im übrigen erfolgt die Wittig-Reaktion in an sich bekannter Weise bei Temperaturen von -30 bis etwa +30°C, vorzugsweise -10°C bis +10°C, insbesondere bei etwa 0°C. Man kann dabei entweder beide Ausgangsverbindungen in dem Lösungsmittel vorlegen und dazu die Base geben oder aber eine Lösung des C₁₅-Triarylphosphoniumsalzes vorlegen, die Base zufügen und erst danach eine Lösung des β-Formyl-crotonsäurealkylesters addieren.

Zur Durchführung einer besonders bevorzugten Form der Wittig-Reaktion wird Lithiumhydroxid in Dimethylformamid (DMF) suspendiert und die Suspension auf 0°C abgekühlt. Zu der abgekühlten Suspension wird zunächst langsam eine Lösung des C₁₅-Triarylphosphoniumsalzes in DMF und danach langsam eine Lösung des β-Formyl-crotonsäureesters in DMF zugefügt. Nach 2 bis 10, vorzugsweise 3 bis 5 stündigem Rühren bei Temperaturen von +10 bis -9°C, vorzugsweise +5 bis -5°C, insbesondere +2 bis -2°C, wird das Reaktionsgemisch mit Eiswasser versetzt. Die Wasser/DMF-Phase wird dann beispielsweise mit einem Heptan/Essigsäureethylestergemisch extrahiert.

Die organische Phase wird mit Wasser gewaschen und bei ca. 40°C vom Lösungsmittel befreit.

Die Base, insbesondere das Lithiumhydroxid, verwendet man hierbei in Mengen von 2 bis 6 Mol, vorzugsweise 3 bis 5 Mol pro Mol des C₁₅-Triarylphosphoniumsalzes.

Das Lösungsmittel, insbesondere das DMF, verwendet man im allgemeinen insgesamt in Mengen von 2 bis 8 Litern (1), vorzugsweise 3 bis 6 1, bezogen auf 1 Mol C₁₅-Triarylphosphoniumsalz.

Als Extraktionsmittel verwendet man mit Vorteil Heptan bzw. ein Gemisch aus Heptan und Essigsäureethylester. Es können aber auch alle anderen mit Wasser nicht mischbaren organischen Lösungsmittel, wie Ether, aliphatische Kohlenwasserstoffe, halogenierte und aromatische Kohlenwasserstoffe zur Extraktion des Retinsäurealkylester-Isomerengemisches verwendet werden.

Die Lösungsmittel, insbesondere das DMF und die Alkanole, bleiben bei dieser Extraktion weitgehend in der wäßrigen Phase und halten auch das bei der Wittig-Reaktion gebildete Triphenylphosphinoxid weitgehend in der wäßrigen Phase zurück.

Bei Verwendung von C₁₅-Triarylphosphoniumhalogeniden hat es sich als vorteilhaft erwiesen, die Wittig-Reaktion mit dem β-Formylcrotonsäurealkylester der allgemeinen Formel II in einem 1,2-Epoxy-alkan mit 3 bis 6 C-Atomen, vorzugsweise in 1,2-Epoxy-butan oder 1,2-Epoxyhexan, und ohne Zusatz einer der obengenannten, für Wittig-Reaktionen üblichen Basen durchzuführen. Die 1,2-Epoxy-alkane spalten dabei aus den C₁₅-Triarylphosphoniumhalogeniden Halogenwasserstoff ab unter Bildung von 2-Halogen-alkanolen und den entsprechenden C₁₅-Triaryl-phosphoranen (vgl. J. Buddrus in Angew. Chem. 80 (1968), Seiten 35 - 36). Letzteres setzt sich dann mit dem β-Formyl-crotonsäurealkylester der allgemeinen Formel II zu dem entsprechenden Retinsäurealkylester um. Das 1,2-Epoxy-alkan wirkt bei dieser Umsetzung also sowohl als Lösungsmittel als auch als Base.

Zur Durchführung dieser Verfahrensvariante werden das C₁₅-Triarylphsphoniumhalogenid und der β-Formyl-crotonsäureester in dem 1,2-Epoxy-alkan suspendiert, die Suspension langsam auf Temperaturen von etwa 35 bis 65°C, vorzugsweise 55 bis 65°C, erwärmt und dann noch 2 bis 24 Stunden, vorzugsweise 4 bis 16 Stunden, bei dieser Temperatur gerührt. Nach Abschluß der Wittig-Reaktion wird das Epoxyalkan aus dem Reaktionsgemisch unter schonenden Bedingungen abdestilliert.

Das 1,2-Epoxy-alkan verwendet man - wie die anderen Lösungsmittel - in Mengen von etwa 2 bis 8, vorzugsweise 3 bis 6 1, bezogen auf 1 Mol C₁₅-Triarylphosphoniumhalogenid.

Die Aufarbeitung des Reaktionsgemisches erfolgt im wesentlichen wie oben für die Wittig-Reaktion in anderen Lösungsmitteln beschrieben.

Einen weiteren großen Vorteil beim erfindungsgemäßen Verfahren erzielt man, wenn man im Reaktionsschritt C
a) den erhaltenen öligen Retinsäurealkylester in einem Propanol oder einem Butanol löst und
b) durch Erwärmen mit einer 10 bis 80 gew.-%igen, vorzugsweise einer 15 bis 60 gew.-%igen, insbesondere einer 20 bis 30 gew.-%igen wäßrigen Lösung eines Alkali- oder Erdalkalihydroxids, vorzugsweise eines Alkalihydroxids, verseift,
c) durch Zugabe einer Mineralsäure zu dem abgekühlten alkalischen Reaktionsgemisch Retinsäure freisetzt und
d) durch Zugabe von Methanol zu der propanolischen oder butanolischen Lösung 9-(Z)-Retinsäure kristallin ausfällt.

Bei dieser Art der Esterspaltung tritt überraschenderweise keine nachteilige Beeinflussung des Isomerenverhältnisses auf. Bei Verwendung von C₃- bis C₉-Alkanolen, vorzugsweise von Propanolen und Butanolen, insbesondere von Isobutanol als Lösungsmittel für die Esterspaltung kann durch Zusatz eines niedrigeren Alkanols, insbesondere von Methanol die 9-(Z)-Retinsäure besonders vorteilhaft in Form hell-oranger Kristalle ausgefällt werden, während all-(E)-Retinsäure oder andere Retinsäureisomere in Lösung gehalten werden. Alternativ kann die Konzentration von 9-(Z)-Retinsäureester in dem Alkanol beim Verseifungsprozess so eingestellt werden, daß direkt eine Kristallisation von 9-(Z)-Retinsäure aus dem Reaktionsansatz heraus auch ohne Zugabe von Methanol erreicht werden kann.

Mit Hilfe des erfindungsgemäßen Verfahrens kann 9-(Z)-Retinsäure erstmals auf relativ einfache Weise und ohne Anwendung aufwendiger chromatographischer Reinigungsmethoden in der Endstufe erhalten werden. Besonders vorteilhaft ist, daß die bei der großtechnischen Herstellung von C₁₅-Triarylphosphoniumsalzen anfallenden Mutterlaugen, die bisher entsorgt werden mußten, genutzt werden können. Erfindungsgemäß ist die Anreicherung des 9-(Z)-C₁₅-Triarylphosphoniumsalzes relativ einfach realisierbar, wodurch eine problemlose Isolierung der 9-(Z)-Retinsäure in der Endstufe des erfindungsgemäßen Verfahrens in dem speziellen Lösungsmittelsystem ermöglicht wird.

Die folgenden Ausführungsbeispiele sollen die Durchführung des erfindungsgemäßen Verfahrens veranschaulichen.

Für die HPLC-Analysen wurden folgende Bedingungen gewählt:
a) C₁₅-Triphenylphosphoniumsalz:
   Nucleosil 120, 7 µm, RP-18, 1 ml/min, 220 nm, Methanol/Wasser= 7:3 + 0,04 % Cetylammoniumbromid, pH= 2,0
b) Retinsäure: Lichrosorb, 5 µm, RP-18, 1,2 ml/min, 352 nm, Methanol/Wasser/Essigsäure= 80:20:0,5

### Beispiel 1

### Herstellung von 9-(Z)-Retinsäure

### A. Anreicherung von 9-(Z)-C₁₅-Triphenylphosphoniumsalz

1 Liter einer Mutterlauge von der Herstellung von 3-Methyl-5-(2,6,6-trimethyl-1-cylcohexen-1-yl)-2,4-pentadienyltriphenylphosphoniumhydrogensulfat (C₁₅-Triphenylphosphoniumsulfat) in Heptan/Isopropanol wurde mit 250 ml Wasser versetzt und intensiv durchmischt. Nach Abtrennung der organischen Phase wurde die wäßrige Lösung in einem Rotationsverdampfer bei 90°C vollständig eingeengt. Man erhielt 91,8 g eines hellbraunen Öls, welches gemäß HPLC-Analyse zu etwa 38 % aus 9-(Z)- und zu 53 % aus all-(E)-C₁₅-Triphenylphosphoniumsulfat bestand. Dieses Öl wurde in 120 ml Isopropanol aufgenommen, wobei eine etwa 50 gew.-%ige Lösung entstand. Anschließend ließ man die Lösung über Nacht im Kühlschrank stehen, filtrierte die ausgeschiedenen Kristalle ab und dampfte die Lösung in einem Rotationsverdampfer ein. Man erhielt 42,2 g eines hellbraunen Öls, welches gemäß HPLC-Analyse zu etwa 65,8 % aus dem 9-(Z)-C₁₅-Triphenylphosphoniumhydrogensulfat und zu 19 % aus all-(E)-C₁₅-Triphenylphosphoniumhydrogensulfat bestand.

### B. Wittig-Reaktion mit β-Formylcrotonsäuremethylester

9 g (0,37 mol) LiOH wurden in 100 ml Dimethylformamid (DMF) suspendiert und unter N₂-Schutzgas auf 0° abgekühlt. Anschließend wurde eine Lösung von 39,6 g (0,0704 mol) des gemäß A. erhaltenen C₁₅-Triphenylphosphoniumsalzes in 100 ml DMF innerhalb von 30 Minuten (min) bei 0°C zu der LiOH-Suspension getropft. Anschließend wurde eine Lösung von 14,5 g (0,113 mol) β-Formyl-crotonsäuremethylester in 70 ml DMF innerhalb von 30 min zugefügt.

Nach 4-stündigem Nachrühren des Reaktionsgemisches bei 0°C wurde mit 400 ml Eiswasser versetzt. Die Wasser/DMF-Phase wurde dann 3 mal mit 180 ml eines Heptan/Essigsäureethylester (2/1)-Gemisches extrahiert. Die erhaltene organische Phase wurde noch 2 mal mit je 100 ml Wasser gewaschen und dann im Rotationsverdampfer eingeengt, wobei 17,85 g eines braungefärbten Öls erhalten wurde.

### C. Isolierung von 9-(Z)-Retinsäure

17,85 g des gemäß B. erhaltenen Retinsäureestergemisches wurde in 50 ml Isobutanol aufgenommen und mit 12,5 g einer 25 gew.-%igen wäßrigen NaOH versetzt und das Reaktionsgemisch zunächst für 75 min. bei 80°C gehalten, dann auf 60°C abgekühlt und mit 38 ml einer 10 gew.-%igen wäßrigen Schwefelsäure versetzt. Nach der Phasentrennung wurde die Isobutanolphase mit 40 ml Methanol versetzt, wobei sofort 9-(Z)-Retinsäure auskristallisierte. Nach der Filtration der Kristalle wurde noch 2 mal mit je 50 ml Methanol (-30°C) gewaschen. Man erhielt 4 g 9-(Z)-Retinsäure. Das entspricht einer Ausbeute von 32 %, bezogen auf in der Mutterlauge der C₁₅-Salz-Produktion enthaltenes 9-(Z)-C₁₅-Triphenylphosphoniumhydrogensulfat.

Die nach der Isolierung von 9-(Z)-Retinsäure anfallende alkoholische Mutterlauge (13,5 g) besteht gemäß HPLC-Analyse noch zu 35 % aus all-(E)-Retinsäure, 17,2 % 9-(Z)-Retinsäure und 34 % unbekannten Retinsäureisomeren.

### D. Umkristallisation von 9-(Z)-Retinsäure

5,2 g einer analog A. bis C. hergestellten 9-(Z)-Retinsäure (Reinheit 94,3 %) wurden in 80 ml eines Isobutanol/Methanol-Gemisches (1/1) für 10 min. bei 80°C gerührt. Anschließend wurde auf 0°C abgekühlt. Innerhalb einer Stunde kristallisierte 9-(Z)-Retinsäure aus, wurde abfiltriert und mit 50 ml Methanol (-30°C) gewaschen. Man erhielt 4,0 g 9-(Z)-Retinsäure in einer Reinheit von 99,5 %.

### Beispiel 2

### A. Anreicherung von 9-(Z)-C₁₅-Triphenylphosphoniumhalogenid

1 Liter einer Mutterlauge von der Herstellung von 3-Methyl-5-(2,6,6-trimethyl-1-cyclohexen-1-yl)-2,4-pentadienyltriphenylphosphoniumchlorid in Heptan/Isopropanol wurde mit 300 ml Wasser versetzt und intensiv durchgemischt. Nach Abtrennen der organischen Phase wurde die wäßrige Lösung in einem Rotationsverdampfer bei 80°C vollständig eingedampft. Man erhielt 102 g eines hellbraunen Öls, welches gemäß HPLC-Analyse zu etwa 42% aus 9-(Z)- und zu 44 % aus all-(E)-C₁₅-Triphenylphosphoniumchlorid bestand. Dieses Öl wurde in 150 ml Isopropanol aufgenommen, wobei eine etwa 50 gew.-%ige Lösung entstand. Anschließend ließ man die Lösung über Nacht im Kühlschrank stehen, filtrierte das auskristallisierte all-(E)-C₁₅-Triphenylphosphoniumchlorid ab und dampfte die Lösung in einem Rotationsverdampfer ein. Man erhielt ein hellbraunes Öl, welches gemäß HPLC-Analyse zu etwa 63% aus 9-(Z)-C₁₅-Triphenylphosphoniumchlorid bestand.

### B. Wittig-Reaktion in 1,2-Epoxy-butan

50,05 g (0,1 mol) des gemäß Beispiel 2A. erhaltenen C₁₅-Triphenylphosphoniumchlorids wurden in 500 ml 1,2-Epoxy-butan suspendiert, diese Suspension bei 25°C mit 14,1 g (0,11 mol) β-Formyl-crotonsäuremethylester versetzt, dann innerhalb von 1 h bis zur Rückflußtemperatur von 65°C erwärmt und schließlich weitere 12 h unter Rückfluß zum Sieden erhitzt. Danach wurde überschüssiges 1,2-Epoxy-butan bei 40°C/150 - 200 mbar abdestilliert und der Rückstand mit 250 ml eines Hexan/Essigsäureethylester-Gemisches (1:1) und 250 ml auf 5°C abgekühltem Wasser versetzt und extrahiert. Die erhaltene organische Phase wurde noch 2 mal mit je 100 ml Wasser gewaschen und dann bei 30°C/20 mbar eingeengt. wobei 19,2 g eines bräunlichen Öls erhalten wurden. Der 9-(Z)-Anteil in dem so erhaltenen Retinsäuremethylestergemisch betrug nach HPLC-Analyse 37 - 41 Gew.-%.

### C. Die Isolierung und Reinigung von 9-(Z)-Retinsäure

erfolgte analog den Beispielen 1 C und 1 D.

### Beispiel 3

### Wittig-Reaktion in 1,2-Epoxy-hexan

25 g (0,046 mol) eines analog Beispiel 2A. erhaltenen C₁₅-Triphenylphosphoniumbromids wurden in 250 ml 1,2-Epoxy-hexan zusammen mit 9,6 g (0,075 mol) β-Formyl-crotonsäuremethylester vorgelegt, das Gemisch innerhalb von 2 h auf 62°C erwärmt und dann noch 10 h bei dieser Temperatur gerührt. Anschließend wurde überschüssiges 1,2-Epoxy-hexan (kp 116 - 120°C) bei 60°C/50 - 100 mbar abdestilliert und der Rückstand mit 100 ml Heptan, 100 ml Essigsäureethylester und 200 ml einer auf 5°C abgekühlten 2 gew.-%igen wäßrigen Sodalösung versetzt und extrahiert. Die erhaltene wäßrige Phase wurde noch 2 mal mit je 150 ml eines Heptan/Essigsäuremethylester-Gemisches(2:1) extrahiert. Die vereinigten organischen Phasen wurden noch 3 mal mit Wasser gewaschen und dann bei 30°C/20 mbar eingeengt. Man erhielt 8,5 g eines bräunlichen Öls, welches analog Beispiel 1 C und 1 D in reine 9-(Z)-Retinsäure überführt wurde.

## Patentansprüche

1. Verfahren zur Herstellung von 9-(Z)-Retinsäure aus Mutterlaugen der technischen Herstellung von C₁₅-Triarylphosphoniumsalzen der allgemeinen Formel I 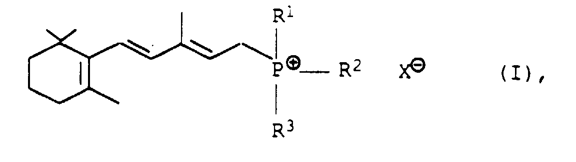 in der R¹ bis R³ für einen Arylrest stehen und X für Halogen oder den Rest (HSO₄) steht, in einem organischen Lösungsmittel, dadurch gekennzeichnet, daß man
A. in den aus den Mutterlaugen durch Extraktion mit Wasser und Einengen der wäßrigen Phase isolierten C₁₅-Triarylphosphoniumsalzen den Anteil an 9-(Z)-C₁₅-Triarylphosphoniumsalzen dadurch anreichert, daß man das ölige C₁₅-Triaryl-phosphoniumsalzgemisch unter Erwärmen in möglich wenig von einem niederen Alkanol löst und das beim Abkühlen auskristallisierende all-(E)-C₁₅-Triarylphosphoniumsalz abtrennt,
B. das erhaltene, an dem 9-(Z)-Isomeren angereicherte C₁₅-Triarylphosphoniumsalz der Formel I in Gegenwart eines Alkali- oder Erdalkalihydroxids, eines Alkali- oder Erdalkaliamids, eines Alkalicarbonats oder von Ammoniak in einem für Wittig-Reaktionen geeigneten Lösungsmittel mit einem β-Formyl-crotonsäurealkylester der allgemeinen Formel II 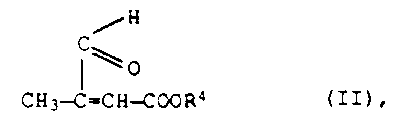 in der R⁴ für eine C₁-C₄-Alkylgruppe steht, umsetzt und
C. das bei der für Wittig-Reaktionen üblichen Aufarbeitung erhaltene obige Retinsäureestergemisch in einem C₃- bis C₉-Alkanol verseift und die erhaltene 9-(Z)-Retinsäure aus der alkanolischen Lösung ggf. durch Zusatz von Methanol kristallin ausfällt, wobei die gebildete all-(E)-Retinsäure und andere unbekannte Retinsäureisomere in der alkanolischen Lösung verbleiben.

2. Verfahren zur Herstellung von 9-(Z)-Retinsäure gemäß Anspruch 1, dadurch gekennzeichnet, daß man im Reaktionsschritt A
das ölige C₁₅-Triarylphosphoniumsalzgemisch in Isopropanol als niederem Alkanol löst.

3. Verfahren gemäß Anspruch 1, dadurch gekennzeichnet, daß man die erhaltene 9-(Z)-Retinsäure anschließend aus einem Butanol/Methanol-Gemisch umkristallisiert.

4. Verfahren zur Herstellung von 9-(Z)-Retinsäure, gemäß Anspruch 1, dadurch gekennzeichnet, daß man im Reaktionsschritt A
den Anteil an dem 9-(Z)-C₁₅-Triarylphosphoniumsalz aus den Mutterlaugen der C₁₅-Triarylphosphoniumsalz-Herstellung dadurch isoliert und anreichert, daß man
a) die Mutterlauge mit Wasser versetzt und die organische Phase abtrennt,
b) die wäßrige Phase unter schonenden Bedingungen einengt,
c) das so erhaltene Öl unter Erwärmen in möglichst wenig Isopropanol löst,
d) das durch Abkühlen auskristallisierende all-(E)-C₁₅-Triarylphosphoniumsalz abtrennt und
e) die erhaltene isopropanolische Lösung unter schonenden Bedingungen einengt.

5. Verfahren zur Herstellung von 9-(Z)-Retinsäure gemäß Anspruch 1, dadurch gekennzeichnet, daß man im Reaktionsschritt B
das an dem 9-(Z)-Isomeren angereicherte C₁₅-Triarylphosphoniumsalz der allgemeinen Formel I in Gegenwart eines Alkali- oder Erdalkalihydroxids eines Alkalicarbonats oder von Ammoniak in Dimethylformamid, einem Alkanol-Dimethylformamid-Gemisch, einem Alkanol-Wasser-Gemisch, in 1,4-Dioxan, Wasser oder in N-Methyl-pyrrolidon als Lösungsmittel mit dem β-Formyl-crotonsäurealkylester der allgemeinen Formel II umsetzt.

6. Verfahren gemäß Anspruch 5, dadurch gekennzeichnet, daß man das an dem 9-(Z)-Isomeren angereicherte C₁₅-Triarylphosphoniumsalz der allgemeinen Formel I mit dem β-Formylcrotonsäurealkylester der allgemeinen Formel II in Dimethylformamid oder 1,4-Dioxan als Lösungsmittel umsetzt.

7. Verfahren zur Herstellung von 9-(Z)-Retinsäure gemäß Anspruch 1, dadurch gekennzeichnet, daß man im Reaktionsschritt B
das an dem 9-(Z)-Isomeren angereicherte C₁₅-Triarylphosphoniumsalz der allgemeinen Formel I, in der R¹ bis R³ für einen Arylrest stehen und X für Halogen steht, in einem 1,2-Epoxyalkan mit 3 bis 6 C-Atomen und ohne Zusatz wesentlicher Mengen einer der für Wittig-Reaktionen üblichen Basen mit dem β-Formyl-crotonsäurealkylester der allgemeinen Formel II umsetzt.

8. Verfahren zur Herstellung von 9-(Z)-Retinsäure gemäß Anspruch 1, dadurch gekennzeichnet, daß man im Reaktionsschritt C
das erhaltene Retinsäureestergemisch in einem Propanol oder einem Butanol verseift und die erhaltene 9-(Z)-Retinsäure aus der propanolischen oder butanolischen Lösung mit Methanol ausfällt.

9. Verfahren zur Herstellung von 9-(Z)-Retinsäure gemäß Anspruch 1, dadurch gekennzeichnet, daß man im Reaktionsschritt C
a) den erhaltenen öligen Retinsäurealkylester in einem Butanol löst und
b) durch Erwärmen mit einer 10 bis 80 gew.-%igen wäßrigen Lösung eines Alkali- oder Erdalkalihydroxids verseift,
c) durch Zugabe einer Mineralsäure zu dem abgekühlten alkalischen Reaktionsgemisch Retinsäure freisetzt und
d) durch Zugabe von Methanol zu der butanolischen Lösung 9-(Z)-Retinsäure kristallin ausfällt.

10. Verfahren zur Anreicherung des 9-(Z)-Anteils in Mutterlaugen der technischen Herstellung von C₁₅-Triarylphosphoniumsalzen der allgemeinen Formel I 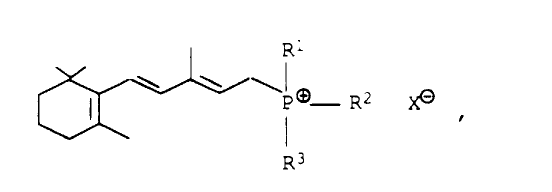 in der R¹ bis R³ für einen Arylrest stehen und X für Halogen oder den Rest (HSO₄) steht, in einem organischen Lösungsmittel, dadurch gekennzeichnet, daß man das aus den Mutterlaugen durch Extraktion mit Wasser und Einengen der wäßrigen Phase isolierte C₁₅-Triarylphosphoniumsalz unter Erwärmen in möglichst wenig von einem niederen Alkanol löst und das beim Abkühlen auskristallisierende all-(E)-Triarylphosphoniumsalz abtrennt.

## Claims

1. A process for preparing 9-(Z)-retinoic acid from mother liquors from the industrial preparation of C₁₅-triarylphosphonium salts of the general formula I 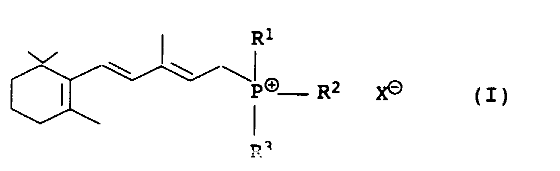 where R¹ to R³ are each aryl, and X is halogen or (HSO₄), in an organic solvent, which comprises
A. increasing the proportion of 9-(Z)-C₁₅-triarylphosphonium salts in the C₁₅-triarylphosphonium salts which have been isolated from the mother liquors by extraction with water and concentration of the aqueous phase, by dissolving the oily C₁₅-triarylphosphonium salt mixture with heating in the minimum amount of a lower alkanol and removing the all-(E)-C₁₅-triarylphosphonium salt which crystallizes out on cooling,
B. reacting the resulting C₁₅-triarylphosphonium salt of the formula I which is enriched in the 9-(Z) isomer, in the presence of an alkali metal or alkaline earth metal hydroxide, of an alkali metal or alkaline earth metal amide, of an alkali metal carbonate or of ammonia, in a solvent suitable for Wittig reactions, with an alkyl β-formylcrotonate of the general formula II 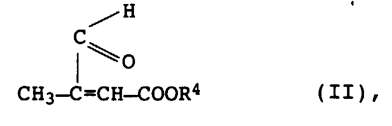 where R⁴ is C₁-C₄-alkyl, and
C. hydrolyzing the above mixture of retinoic esters, which has been obtained by the conventional workup for Wittig reactions, in a C₃-C₉-alkanol and precipitating the resulting 9-(Z)-retinoic acid as crystals from the alkanolic solution where appropriate by adding methanol, with the all-(E)-retinoic acid which is formed and other unknown retinoic acid isomers remaining in the alkanolic solution.

2. A process for preparing 9-(Z)-retinoic acid as claimed in claim 1, wherein in step A the oily C₁₅-triarylphosphonium salt mixture is dissolved in isopropanol as lower alkanol.

3. A process as claimed in claim 1, wherein the resulting 9-(Z)-retinoic acid is subsequently recrystallized from a butanol/methanol mixture.

4. A process for preparing 9-(Z)-retinoic acid as claimed in claim 1, wherein in step A the proportion of 9-(Z)-C₁₅-triarylphosphonium salt from the mother liquors from the C₁₅-triarylphosphonium salt preparation is isolated and increased by
a) adding water to the mother liquor and separating off the organic phase,
b) concentrating the aqueous phase under mild conditions,
c) dissolving the resulting oil with heating in the minimum amount of isopropanol,
d) separating off the all-(E)-C₁₅-triarylphosphonium salt which crystallizes out on cooling, and
e) concentrating the resulting isopropanolic solution under mild conditions.

5. A process for preparing 9-(Z)-retinoic acid as claimed in claim 1, wherein in step B the C₁₅-triarylphosphonium salt of the general formula I, which has been enriched in the 9-(Z) isomer, is reacted in the presence of an alkali metal or alkaline earth metal hydroxide, of an alkali metal carbonate or of ammonia, in dimethylformamide, an alkanol/dimethylformamide mixture, an alkanol/water mixture, in 1,4-dioxane, water or n-methylpyrrolidone as solvent with the alkyl β-formylcrotonate of the general formula II.

6. A process as claimed in claim 5, wherein the C₁₅-triarylphosphonium salt of the general formula I which is enriched in the 9-(Z) isomer is reacted with the alkyl β-formylcrotonate of the general formula II in dimethylformamide or 1,4-dioxane as solvent.

7. A process for preparing 9-(Z)-retinoic acid as claimed in claim 1, wherein in step B the C₁₅-triarylphosphonium salt of the general formula I which is enriched in the 9-(Z) isomer and in which R¹ to R³ are each aryl and X is halogen is reacted in a 1,2-epoxyalkane with 3 to 6 carbon atoms and without adding significant amounts of one of the bases conventional for Wittig reactions with the alkyl β-formylcrotonate of the general formula II.

8. A process for preparing 9-(Z)-retinoic acid as claimed in claim 1, wherein in step C the resulting retinoic ester mixture is hydrolyzed in a propanol or a butanol and the resulting 9-(Z)-retinoic acid is precipitated from the propanolic or butanolic solution with methanol.

9. A process for preparing 9-(Z)-retinoic acid as claimed in claim 1, wherein in step C
a) the resulting oily alkyl retinoate is dissolved in a butanol, and
b) hydrolyzed by heating with a 10 - 80% by weight aqueous solution of an alkali metal or alkaline earth metal hydroxide,
c) retinoic acid is liberated by adding a mineral acid to the cooled alkaline reaction mixture, and
d) 9-(Z)-retinoic acid is precipitated as crystals by adding methanol to the butanolic solution.

10. A process for increasing the 9-(Z) proportion in mother liquors from the industrial preparation of C₁₅-triarylphosphonium salts of the general formula I 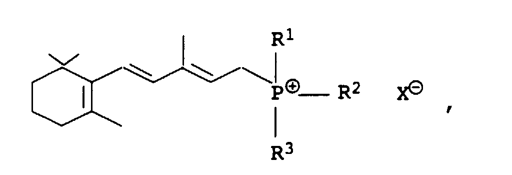 where R¹ to R³ are each aryl and X is halogen or (HSO₄), in an organic solvent, which comprises dissolving the C₁₅-triarylphosphonium salt which has been isolated from the mother liquors by extraction with water and concentration of the aqueous phase, with heating in the minimum amount of a lower alkanol, and separating off the all-(E)-triarylphosphonium salt which crystallizes out on cooling.

## Revendications

1. Procédé pour la préparation d'acide 9-(Z)-rétinique à partir de liqueurs mères provenant de la préparation industrielle de sels de C₁₅-triarylphosphonium de formule générale I 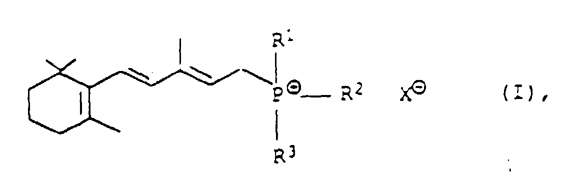 où R¹ à R³ désignent un reste aryle et X représente un halogène ou le reste (HSO₄), dans un solvant organique, caractérisé par le fait que
A. dans les sels de C₁₅-triarylphosphonium isolés à partir des liqueurs mères par extraction avec de l'eau et concentration de la phase aqueuse, on enrichit la fraction de sels de 9-(Z)-C₁₅-triarylphosphonium en dissolvant le mélange huileux de sels de C₁₅-triaryl-phosphonium, en chauffant, dans le moins possible d'un alcanol inférieur et on sépare le sel de tout-(E)-C₁₅-triarylphosphonium qui cristallise par refroidissement,
B. on fait réagir le sel de C₁₅-triarylphosphonium de formule I obtenu, enrichi en l'isomère 9-(Z), en présence d'un hydroxyde alcalin ou alcalino-terreux, d'un amide alcalin ou alcalino-terreux, d'un carbonate alcalin ou d'ammoniac, dans un solvant approprié pour des réactions de Wittig, avec un ester alkylique d'acide β-formylcrotonique de formule générale II 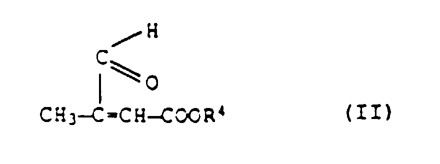 où R⁴ désigne un groupe alkyle en C₁-C₄, et
C. on saponifie le mélange d'acides rétiniques susdit, obtenu lors du traitement classique pour des réactions de Wittig, dans un alcanol en C₃ à C₉, et on précipite sous forme cristallisée l'acide 9-(Z)-rétinique obtenu à partir de la solution alcanolique, éventuellement avec addition de méthanol, tandis que l'acide tout-(E)-rétinique formé et d'autres isomères inconnus de l'acide rétinique restent dans la solution alcanolique.

2. Procédé pour la préparation d'acide 9-(Z)-rétinique selon la revendication 1, caractérisé par le fait que, dans l'étape réactionnelle A,
on dissout le mélange huileux de sel de C₁₅-triarylphosphonium dans de l'isopropanol en tant qu'alcanol inférieur.

3. Procédé selon la revendication 1, caractérisé par le fait qu'on recristallise ensuite l'acide 9-(Z)-rétinique obtenu, dans un mélange de butanol/méthanol.

4. Procédé pour la préparation d'acide 9-(Z)-rétinique selon la révendication 1, caractérisé par le fait que, dans l'étape réactionnelle A,
on isole et enrichit la fraction de sel de 9-(Z)-C₁₅-triarylphosphonium à partir des liqueurs mères de sel de C₁₅-triarylphosphonium en
a) ajoutant de l'eau à la liqueur mère et séparant la phase organique,
b) concentrant la phase aqueuse dans des conditions douces,
c) dissolvant l'huile ainsi obtenue, en chauffant, dans le moins possible d'isopropanol,
d) séparant le sel de tout-(E)-C₁₅-triarylphosphonium qui se sépare par cristallisation au refroidissement et
e) concentrant la solution isopropanolique obtenue, dans des conditions non perturbatrices.

5. Procédé pour la préparation d'acide 9-(Z)-rétinique selon la revendication 1, caractérisé par le fait que, dans l'étape réactionnelle B,
on fait réagir le sel de C₁₅-triarylphosphonium de formule générale I enrichi en isomère 9-(Z) en présence d'un hydroxyde alcalin ou alcalino-terreux, d'un carbonate alcalin ou d'ammoniac, dans du diméthylformamide, un mélange d'alcanol-diméthylformamide, un mélange d'alcanol-eau, dans du 1,4-dioxanne, dans de l'eau ou dans de la N-méthylpyrrolidone en tant que solvant, avec l'ester alkylique d'acide β-formyl-crotonique de formule générale II.

6. Procédé selon la revendication 5, caractérisé par le fait qu'on fait réagir le sel de C₁₅-triarylphosphonium de formule générale I enrichi en l'isomère 9-(Z) avec l'ester alkylique d'acide β-formyl-crotonique de formule générale II dans du diméthylformamide ou du 1,4-dioxanne en tant que solvant.

7. Procédé pour la préparation d'acide 9-(Z)-rétinique selon la revendication 1, caractérisé par le fait que, dans l'étape réactionnelle B,
on fait réagir le sel de C₁₅-triarylphosphonium de formule générale I enrichi en isomère 9-(Z), dans lequel R¹ à R³ désignent un reste aryle et X représente un halogène, dans un 1,2-époxy-alcane ayant 3 à 6 atomes de carbone et sans addition de quantités notables d'une base usuelle pour les réactions de Wittig, avec l'ester alkylique d'acide β-formyl-crotonique de formule générale II.

8. Procédé pour la préparation d'acide 9-(Z)-rétinique selon la revendication 1, caractérisé par le fait que, dans l'étape réactionnelle C,
on saponifie le mélange d'esters d'acide rétinique obtenu dans un propanol ou un butanol et on précipite l'acide 9-(Z)-rétinique obtenu, dans la solution propanolique ou butanolique, avec du méthanol.

9. Procédé pour la préparation d'acide 9-(Z)-rétinique selon la revendication 1, caractérisé par le fait que, dans l'étape réactionnelle C,
a) on dissout l'ester alkylique d'acide rétinique huileux obtenu dans un butanol et
b) on saponifie par chauffage avec une solution aqueuse à 10 à 80 % en poids d'un hydroxyde alcalin ou alcalino-terreux,
c) on libère de l'acide rétinique par addition d'un acide minéral au mélange réactionnel alcalin refroidi et
d) on précipite de l'acide 9-(Z)-rétinique cristallisé par addition de méthanol à la solution butanolique.

10. Procédé pour l'enrichissement de la fraction 9-(Z) dans des liqueurs mères de préparation industrielle de sels de C₁₅-triarylphosphonium de formule générale I 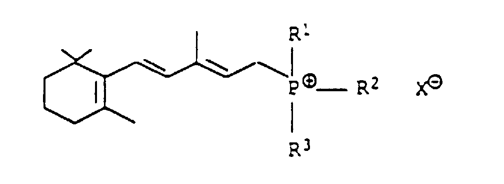 où R¹ à R³ désignent un reste aryle et X représente un halogène ou le reste (HSO₄), dans un solvant organique, caractérisé par le fait qu'on dissout le sel de C₁₅-triarylphosphonium isolé à partir des liqueurs mères par extraction avec de l'eau et concentration de la phase aqueuse, en chauffant, dans le moins possible d'un alcanol inférieur et on sépare le sel de tout-(E)-C₁₅-triarylphosphonium qui cristallise par refroidissement.
